# EUROPEAN PATENT APPLICATION

(11) **EP 1 911 834 A1**
(43) Date of publication of application: **16.04.2008**
(21) Application number: 07024724.2
(22) Date of filing: 24.12.2002
(51) Int. Cl.: C12M 3/00, G01N 35/10, B01L 3/00

(54) **Analytical device for cell cultures**

(62) Divisional of application: 02808318.6
(71) Applicant: Humanitas Mirasole S.p.A., 20089 Rozzano (Milano) (IT)
(72) Inventor: Parmeggiani, Luciano, 20089 Rozzano Milano (IT); Dioguardi, Nicola, 20089 Rozzano Milano (IT); Grizzi, Fabio, 20089 Rozzano Milano (IT)
(74) Representative: Long, Giorgio

(57) **Abstract**

The present invention relates to an analytical device (1) for cell cultures comprising a container (4) for cell cultures, a reservoir (2) for the storage of fresh culture medium for said cell cultures, pumping means (3) suitable to introduce said fresh culture medium into said container and to remove metabolised culture medium from the cell cultures of said of said container and a depositing device (5) suitable for depositing the metabolised culture medium removed from said container by means of said pumping means onto an analytical support (11).

## Description

The present invention relates to an analytical device for cell cultures, and a cell culture analytical method. In addition, the present invention relates to a cell culture container for use in said analytical device and method.

The current methods of *in* vitro cell cultivation in general use physical systems known by the term "closed systems". With this term is intended, in the present description, a system fundamentally comprised of a closed container inside of which has been placed a culture medium comprising a determined quantity of cells. In particular, said container must be open to allow access to the cell culture for removing a sample for analysis or to allow the replenishment of the cells with the nutritional substances necessary for their survival.

The culture medium thus remains inside the container and undergoes changes of its constituents with time (such as for example amino acids, mineral salts, vitamins and antibiotics) and/or of the relative concentrations due to their being metabolised by the cells contained in it. The studies which refer to the cellular metabolism in these closed systems involve therefore removing appropriate amounts of culture medium from the container at well determined times. Successively, the samples, in order to be analysed, must be manipulated so as to make them available for the type of analysis desired.

Generally, said samples are made by a pipette worked by an operator who extracts from the culture container a quantity of the desired sample and deposits it onto an appropriate analytical support or into a test tube.

Regarding liquid culture media, a well known type of container used is represented by the "flask". "Flasks" are usually layered in transparent plastic materials, substantially parallelepiped in shape and having on one wall an aperture fitted with a protruding collar, a closing lid being reversibly screwed thereto.

Usually, therefore, the above mentioned samplings must be made by appropriate instruments, such as the above mentioned pipettes, which must be introduced into the container through the opening of the closing lid. Frequently, in addition, it is necessary to operate in sterile environments or however still use sterile instruments or at least uncontaminated. In addition, it is not rarely necessary to use new instruments or clean pipette tips for every sample removal.

In addition, as mentioned previously, the samplings are made at determined time intervals.

From the description, it is clear that the analytical methods of the known technique first of all require a certain number of manual operations and secondly, said manual operations must be performed by a specialised expert at pre-established times.

All that must obviously take place paying particular attention to not contaminate the cell culture so as not to alter the results of the analyses.

Consequently, the problem at the heart of the present invention is that of providing an analytical device for cell cultures which allows for the above mentioned inconveniences caused by a cellular analysis method developed in a closed system to be overcome.

This problem is solved by a cell culture analytical system as claimed in the attached independent claim.

A second object of the present invention is then that of providing a cell culture analytical method which solves the problems associated with the closed system method.

A further object of the invention is that of providing a cell culture container for use in a device and method as above.

The characteristics and the advantages of the invention will be now described in the following with reference to the attached figures referring to a non limiting example of an embodiment, in which:
- Figure 1 schematically represents a cell culture analytical device according to the invention;
- Figure 2 represents a perspective view of a cell culture container according to the invention.

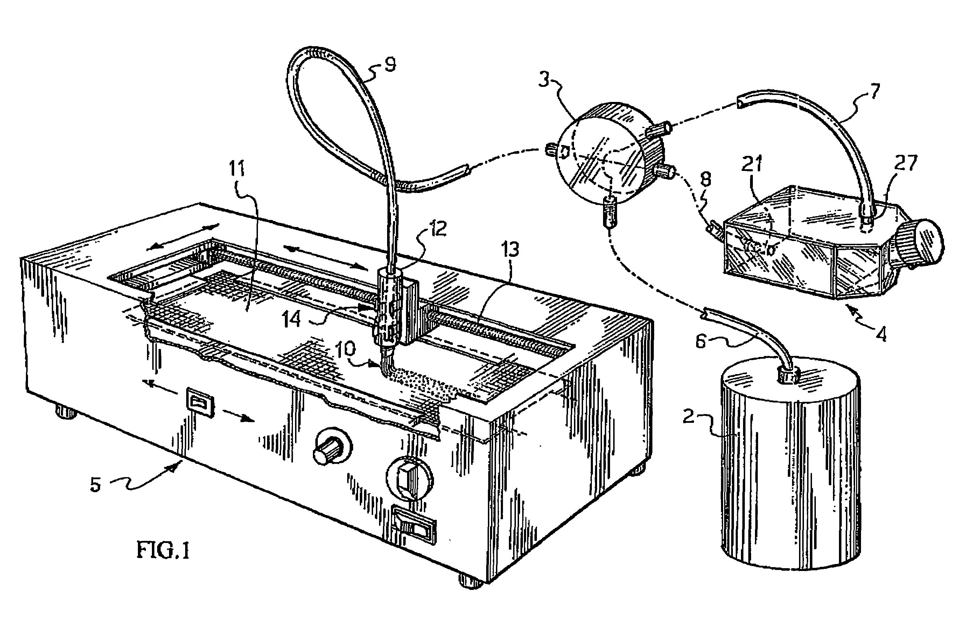

With reference to figure 1, the reference number 1 indicates in general a cell culture analytical device according to the invention. It should be noted that the analytical device 1 is globally represented schematically to allow the understanding of its functioning.

Said analytical device 1 comprises a reserve tank 2, a pump 3, a container 4 and a depositing device 5.

The tank 2, schematically represented by a rectangle in figure 1, consists of any type of container suitable for containing cell culture medium.

Usually, different culture media suitably adapted for every cell type normally used in the sector of *in vitro* culture are commercially available. Alternatively, said media can be prepared by the expert in the field according to particular needs or preferences.

Preferably, the tank 2 can be represented by a bottle of liquid medium or by a conventional flask containing a solution of amino acids, mineral salts, vitamins, antibiotics and other minor components.

In addition, the tank 2 can be maintained at a desired temperature by suitable conventional means of heating such as for example a thermostatic water bath.

The tank 2 is connected to a pump 3 through a tube 6 which allows the pump to suck fresh culture medium from the inside of said tank and to send it to a container 4 through a second connecting tube 7. In this way a first unidirectional closed circuit is formed from the tank 2 to the container 4.

The pump 3 can be of any type commercially available and suitable for transferring liquid cell culture medium from one container to another such as for example a vacuum pump. Preferably, the pump 3 is a peristaltic pump which is calibrated so as to transfer a determined quantity of fresh medium from the tank 2 to the container 4 per unit of time according to the type of cell culture and the type of analyses to be performed. These adjustments however are at the discretion of the expert in the field in as much as they depend on the metabolic profile of the cell culture to be analysed.

In particular, it is to be noted that the use of a peristaltic pump advantageously allows one to obtain a continuous flow of constant delivery capacity over time. The pump 3 is further connected to the container 4 by means of a third tube 8 which allows the removal from said container of the culture medium metabolised by the cells and which must be analysed.

A fourth tube 9 then connects the pump 3 to means of depositing 10 suitable for continuously depositing the culture medium removed from the container 4 onto a support 11 appropriately selected on the basis of the type of analysis to be performed and the type of cell culture used.

Consequently, the tube 8, the pump 3 and the tube 9 represent a second closed circuit, separate and parallel to the first circuit.

The means of depositing 10 can be represented by any instrument suitable for depositing continuously or semi-continuously onto an analytical support, the cell culture medium removed from the container 4. By the term semi-continuously is intended for example depositing dropwise.

In other words, the means of depositing 10 are such as to constantly release the culture medium uniformly distributed, continuously or semi-continuously on a support 11. Said means can comprise for example brushes, pens, felt tips, ink pens (rapidograf®, rotring®).

Amongst the above mentioned means, these preferred are the brushes in as much as they allow one to leave an optimal quantity of culture medium to analyse depositing the culture medium in the form of a thin film. In addition, the hairs which make them, natural or synthetic, can bend without damaging even the most delicate of supports.

Particularly preferred are the natural hair brushes such as, for example, marten hair and ox hair. The length of such hairs can vary according to each case and in general can range from 4 to 10 mm, preferably 8 mm for the ox hairs and 5 mm for the marten hairs.

As mentioned previously, the deposit can therefore be made continuously if the brushes receive a continuous flow, or it can be made in a semi-continuous manner in the case in which the culture medium arrives at the brushes in a dropwise manner preferably maintaining the brush always impregnated with said medium.

The means of deposition 10 are reversibly associated with a depositing device 5, represented schematically in figure 1, as will now be described in detail.

The depositing device 5 comprises the above mentioned support 11 and the moving means 13 suitable for moving the above mentioned means of deposition 10 above said support so as to leave a determined amount of the substance to be analysed along the selected route.

In particular, the moving means 13 are preferably constructed, but not limited to, an Archimedean screw which is operated for example by a conventional type electric motor not shown in figure 1. With said moving means 13 are associated the means of depositing 10 for example by a cursor (not shown).

In addition, the cursor of the moving means 13 is fitted with clamping means 14 suitable to reversibly hold the means of depositing 10 during their movement.

The clamping means 14 can be constituted for example by forceps suitable for gripping a cylindrical support 12 by the end portion of the tube 9 with which are associated the above mentioned means of depositing 10, as schematically represented in figure 1, or directly by the end portion of the tube 9 or still the brush itself. The support 11 is generally constituted by a sheet of material suitable for fixing an amount of culture medium which must be analysed. Amongst the materials normally used for this purpose are sheets or membranes of nitrocellulose or nylon for example.

Obviously, the expert in the field will be able to select which is the support suitable for the type of cell culture medium to analyse taking into consideration also the type of analysis to be performed.

The support 11 can be held in position under the means of depositing 10 in different ways. For example, it can be envisaged to hold the support suspended underneath said means of depositing reversibly anchoring it along its outermost edges in a sort of sandwich.

In other words, the support 11 can be placed on a rigid plane for example or, preferably, only its outermost edge can rest on a metal plate on which magnets are applied.

The depositing device 5 comprises in a addition a conventional switch/adapter to allow the working of the Archimedean screw in one direction of rotation and in the opposite direction thus allowing the movement of the means of depositing 10 alternatively in one or the other direction along said screw.

The speed of rotation of the Archimedean screw can then be regulated by a conventional speed regulator, not shown, so as to change the speed of movement of the means of depositing 10 on the support 11 on the basis of the particular requirements dictated by the operating conditions.

In particular, the travelling times of the means of depositing, for a 40 cm support, could generally vary between 1 and 5 hours.

Additionally, the analytical device 5 can optionally comprise a timer with an exclusion switch.

The container 4 can be of any type of container normally used for in vitro cell culture such as, for example, plates and "flasks".

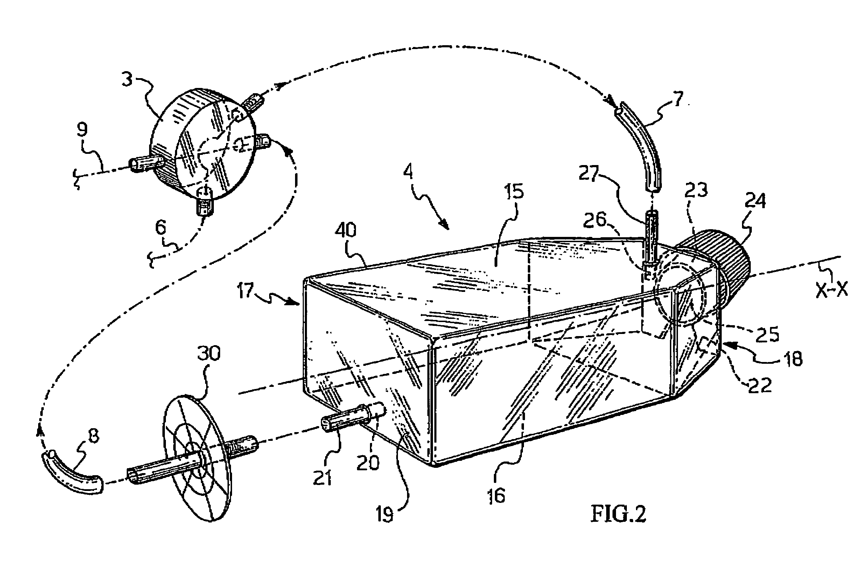

In particular, the container 4 represented in figure 2 consists of a flask which has been advantageously modified such that to allow it to be used in an analytical device in agreement with the invention or however in an analytical method according to the invention.

In detail, the flask comprises a body 40 having a substantially parallelepiped shape which extends along a longitudinal axis X-X. The body 40 presents an upper face 15, a lower face 16, a first end 17 and a second end 18.

The first end 17 is fitted with a substantially planar face 19, rectangular in shape, crossed by and located perpendicularly to the X-X axis.

Advantageously, the face 19 is fitted with a through hole 20 preferably in the vicinity of the upper face 15 of the flask 4. Said hole is communicated to a tube 21 protruding from said face towards the outside.

The second end 18 is tapered towards the outside of the flask 4 or, in other words, has a substantially pyramidal shaped trunk with a smaller base 22 turned towards the outside.

Said smaller base 22 is fitted with a circular opening 25 from the edge of which a cylindrical hollow neck 23 extends, still towards the outside, generally slightly sloped upwards to facilitate the filling or the emptying of the flask 4 and at the same time avoiding the accidental spillage of the liquid when the flask lies horizontally resting on the lower face 16.

Usually, then, on said neck 23 is reversibly screwed a closing lid 24. Said lid can be blank bottomed or can have holes covered by an appropriate conventional membrane (not shown) which allows the exchange of gas between the outside and the inside of the flask 4 but not the loss of the contents or its contamination.

In addition, the upper face 15 of the flask 4 is fitted with a through hole 26 from which extends a tube 27 towards the outside of the flask 4.

Preferably, said hole 26 and said tube 27 are located level with said second end 18.

The functioning of the cell culture analytical device 1 according to the present invention will now be briefly described in the following.

The pump 3 is connected, as previously described, to the flask 4, containing a determined quantity of liquid culture medium inoculated with the desired cells, so that the tube 7 is connected with the tube 27 of the flask and the tube 8 is connected with the tube 21 of the flask.

In turn, the pump 3 is connected to the fresh culture medium reservoir 2 through the above mentioned tube 6 and to the means of deposition 10 through the additional tube 9.

At this point, the pump 3 connects and supplies two separate circuits in which the first circuit starts from the reservoir 2, passes through the pump 3 and ends in the container 4 and the second circuit however starts from the container 4, still passing through the pump 3 without interfering with the first circuit and ends at the means of deposition 10.

In this way, the flask 4 can receive fresh culture medium from the reservoir 2 continuously whilst still continuously unloading the culture medium metabolised by the cells present in it, through the use of a single pump 3.

In particular, it is to be noted that the two circuits just described are controlled by a single mechanical device which allows the regulation of the flow rate of the fresh culture medium entering and exiting from the flask 4 in perfect synchronisation. In other words, the amount of fresh medium placed in the flask 4 per unit of time is always equal to the amount of medium metabolised by the cells in culture removed from the same flask.

This system advantageously allows one to maintain the flow rate of culture medium which is deposited on the analytical support 5 constant.

In addition, the analytical device according to the present invention allows the passage from a "closed system" according to the prior art to an "open system", as will be herein better described in the following.

A further object of the present invention is an analytical method for cell cultures which allows the exploitation of an "open system".

By the term "open system" is intended a system fundamentally constituted by a closed container inside of which is deposited a cell culture medium comprising a determined quantity of cells. In particular, the container can allow the removal of a culture sample or the culture medium for analysis without the need to open the container and to perform said removal manually through an instrument worked by an operator.

Said analytical method involves the continuous removal of an amount of cell culture or of culture medium for analysis from a cell culture container through automated means and the depositing of said amount continuously onto an appropriate analytical support.

In particular, the continuous removal of a cell culture sample for analysis takes place by the above mentioned peristaltic pump 3, i.e. an automated pumping means which once started does not require further intervention from a manual operator. The pump 3 delivery will be calibrated according to the type of medium and/or cells cultivated not least on the type of analysis to perform. In any case, these conditions will be selected each time by the expert in the field on the basis of the needs of the case.

In addition, the removal takes place through a closed circuit which, as described previously, starts from the container, passes through the pump and ends on the support through means of deposition.

The deposition stage is preferably realised by means of deposition such as for example the above mentioned brushes 10. In addition, said stage takes place with continuous movement to allow for uniform distribution and to avoid overlapping of the culture medium on the analytical support along a route, that can be for example rectilinear.

The deposition stage depends on some factors such as for example the pressure of the fluid exiting from the means of deposition, which, in turn determines the speed of the continuous flow of said fluid, the type of culture and the analyses to be performed, the type of means of deposition.

In general, regarding the means of deposition, it can be said that in the case in which brushes are used, for a total length of the resting hairs from 4 to 10 mm, the length in use can vary from between 3 to 7 mm.

In other words, when the brush comes into contact with the analytical support for depositing the culture medium, it will undergo flexing of its hairs reducing their distance between the point of origin and the surface of the support. This phenomenon will determine the depositing pressure of the culture medium necessary for obtaining a film on the support sufficient to perform the analyses required.

In addition, the method of depositing will depend on the type of analytical support, on the properties of the culture medium and on the speed of movement of the brush, on its turn determined by the speed of rotation of the Archimedean screw or other moving means.

All the conditions listed herein are however within the skills of the expert in the field.

The analytical method according to the invention can also comprise a supplying/compensation stage of the culture medium metabolised by the cells and removed during the sampling stage. This step consists in that a determined quantity of fresh medium is removed from a reservoir such as that previously described and placed into the cell culture container.

In other words, the supplying/compensation stage allows to maintaining a constant volume of culture medium from which the cells can continuously draw the fresh substances necessary for the maintenance of their normal vitality.

In particular, the supplying/compensation stage is performed by the feeding means such as for example the same peristaltic pump described above or other similar means.

In any case, it is preferable that the supply/compensation stage is coordinated and regulated with the metabolised medium sample removal stage so as that the amount of culture medium which is removed from the container for analysis is substantially compensated at the same time by an equal amount of fresh medium always added to the container.

As previously described, a peristaltic pump connected in series with the reservoir and with the container can carry out the above mentioned function.

Preferably, both the container and the reservoir are warmed to a temperature of between 35° to 40°C, still more preferably at 37°C, by for example, a thermostatic water bath.

The method of analysis can in addition include a continuous filtration step of the culture medium exiting from the culture container to avoid any eventual loss of the cells which are found in suspension or contamination of the culture medium to be analysed by non relevant elements.

The filtration can be performed by means of filters, such as that schematically represented in figure 2 and indicated by the reference number 30, commonly used in the sector to obtain for example contaminant free solutions. Obviously, the expert in the sector will be able to select the most appropriate filter according to for example the type of cells, the culture medium, and the outlet flow rate.

The types of analyses that can be advantageously performed in accordance with the method in the present invention are preferably targeted towards analysing the continuous secretion of biochemical metabolites or cellular "uptake".

According to what has been described, the advantages provided by the analytical device, by the method and by the container according to the invention appear numerous.

First of all, the system used here is "open" in type or, as previously stated, a system that allows operation with the minimum of intervention from an operator.

In fact, since the container 4 is connected through the pump 3 both to the fresh medium reservoir 2 and to the means of depositing 10, there is no need for a technician to open the container 4 and introduce an instrument to remove a quantity of medium to analyse nor even open the container to introduce new fresh culture medium to guarantee nutritional substances for the cells in culture. This type of system is typical of the above mentioned "closed systems".

In addition, the removal of culture medium is advantageously performed continuously and in an absolutely regular manner since it is completely automated.

On the contrary, when operating in a "closed system", the removal of the samples can take place only at intervals of time and, in addition, with a greater possibility of errors of the quantity removed determined by a manually operated instrument.

Furthermore the risks of contamination, with the system of the invention are at least reduced to a minimum if not indeed eliminated.

Furthermore, the system just described is absolutely optimal from the point of view of the maintenance of cellular homeostasis inside the container.

In turn, the working conditions result in the attainment of a more accurate analysis of the evolution of cellular metabolism.

In addition, automation allows for the direct depositing onto an appropriate analytical support the substance which must be then subjected to the analysis without the addition of an additional operating step on the part of a technician.

As can be appreciated from that described, the analytical device, the method and the container for cell cultures according to the invention allow to satisfy the requirements which have been referred to in the introductory section of the present description, and at the same time to overcome the inconveniences presented by analytical devices known in the art.

Obviously, one skilled in the art, with the aim of satisfying contingent and specific needs, can effect numerous modifications and variations to the device and to the analytical method described above, all of them included within the scope of the invention as defined in the following claims.

## Claims

1. An analytical device (1) for cell cultures comprising a container (4) for cell cultures, a reservoir (2) for fresh culture medium for said cell cultures, a means of pumping (3) suitable for introducing said fresh culture medium into said container by means of a first unidirectional closed circuit (6, 7) and for removing metabolised culture medium from the cell cultures of said container by means of a second closed circuit (8, 9) separate and parallel to the first circuit and a depositing device (5) suitable for depositing the metabolised culture medium removed from said container by means of said pumping means onto an analytical support (11).

2. The analytical device (1) according to claim 1, in which said reservoir (2) is represented by a bottle or flask containing a solution comprising aminoacids, mineral salts, vitamins and antibiotics.

3. The analytical device (1) according to claims 1 or 2 in which, said means of pumping (3) comprise means which create a continuous or semi-continuous flow of constant delivery over time.

4. The analytical device (1) according to claim 3, in which said means of pumping (3) are a vacuum pump or a peristaltic pump.

5. The analytical device (1) according to any of the claims from 1 to 4, in which said depositing device (5) comprises means of depositing (10) suitable to deposit continuously or semi-continuously said cell culture medium onto said support (11).

6. The analytical device (1) according to claim 5, in which said means of depositing (10) comprise pens, marker pen tips, ink pens and brushes.

7. The analytical device (1) according to claim 6, in which said brushes are selected from synthetic hair and natural hair brushes, said natural hairs being from ox or marten.

8. The analytical device (1) according to claim 7, in which the length of the hairs is comprised of between 4 and 10 mm, preferably 8 mm for the ox hairs and 5 mm for the marten hairs.

9. The analytical device (1) according to any of the claims from 5 to 8, in which said depositing device (5) comprises in addition moving means (13) suitable to move said means of depositing (10) on said support (11).

10. The analytical device (1) according to claim 9, in which said moving means (13) comprise an Archimedean screw.

11. The analytical device (1) according to any of the claims from 5 to 10, comprising in addition clamping means (14) suitable for operationally holding said means of depositing (10) on said support (11).

12. The analytical device (1) according to claim 11, in which said clamping means (14) comprise at least one elastic forceps associated with said moving means (13).

13. The analytical device (1) according to any of the claims from 1 to 12, in which said support (11) is selected from the group consisting of nitrocellulose or nylon sheets or membranes.

14. An analytical method for cell cultures comprising the continuous removal of an amount of cell culture or of cell medium to analyse from a cell culture container by automated means and the continuous deposit of said amount on an appropriate analytical support.

15. The analytical method according to claim 14, in which said removal takes place by automated means.

16. The analytical method according to claim 15, in which said automated means is selected from a vacuum pump and a peristaltic pump.

17. The analytical method according to any of the claims from 14 to 16, in which said removal takes place in a closed circuit starting from said container up to said support through said automated means.

18. The analytical method according to any of the claims from 14 to 17, in which said deposition step takes place continuously and with continuous movement to allow uniform distribution on said analytical support.

19. The analytical method according to claim 18, in which said deposition takes place by means of deposition selected from brushes, marker pen tips, pens and ink pens.

20. The analytical method according to any of the claims from 14 to 19, comprising in addition supplying/compensation of the metabolised cell culture medium removed from the container.

21. The analytical method according to claim 20, in which the supplying/compensation comprises the removal of a determined quantity of fresh medium from a reservoir and its transfer into said cell culture container.

22. The analytical method according to claims 20 or 21, in which the supplying/compensation takes place by pump feeding means.

23. The analytical method according to claim 22, in which said pump feeding means comprise the same peristaltic pump as in claim 16.

24. The analytical method according to any of the claims from 20 to 23, in which the supplying/compensation is coordinated with the removal from the container.

25. The analytical method according to claim 24, in which the peristaltic pump is connected in series with the reservoir and with the container.

26. The analytical method according to any of the claims from 21 to 25, in which the reservoir and the container are warmed to a temperature comprised of between 35° and 40°C, preferably 37°C.

27. A container (4) suitable for cell cultures comprising an entry opening (27) for the supply of fresh culture medium and an exit opening (20) for the removal of metabolised culture medium.

28. The container (4) according to claim 27, comprising a body (40) provided with an upper face (15), a lower face (16), a first end (17) and a second end (18), **characterized in that** said first end comprises a face (19) with a through hole (20) suitable to be sealingly engaged with a tube (21) communicated to the outside of said container, and that said upper face comprises a through hole (26) suitable to be sealingly engaged with a tube (27) communicated to the outside.

29. The container (4) according to claims 27 or 28, comprising in addition a neck (23) reversibly closable with a lid (24).

30. The container (4) according to any of the claims 28 and 29, comprising in addition a filter which is applicable to the hole (20) or to the tube (21).

31. The container (4) according to any of the claims from 27 to 30, in which said container is a flask for cell cultures having a parallelepiped shape which extends longitudinally along an X-X axis.
